# EUROPEAN PATENT APPLICATION

(11) **EP 4 497 371 A1**
(43) Date of publication of application: **29.01.2025**
(21) Application number: 22933790.2
(22) Date of filing: 29.12.2022
(51) Int. Cl.: A61B 3/12, A61B 3/00, G06T 7/11, G06T 7/90, G06T 5/00, A61B 5/00, G16H 50/20

(54) **METHOD AND IMAGE PROCESSING DEVICE FOR STANDARDIZING FUNDUS IMAGE ON BASIS OF ARTERY AND VEIN**

(30) Priority: 22.03.2022 KR 20220035220
(71) Applicant: Samsung Life Public Welfare Foundation, Seoul 04348 (KR)
(72) Inventor: KANG, Se Woong, Seoul 06351 (KR); KIM, Yun Taek, Seoul 06597 (KR)
(74) Representative: Ostertag & Partner Patentanwälte mbB
(86) International application number: PCT/KR2022/021570
(87) International publication number: WO 2023/182622

(57) **Abstract**

A method of standardizing a fundus image on the basis of an artery and a vein includes receiving, by an image processing device, an initial fundus image, determining, by the image processing device, reference regions in the initial fundus image, and correcting, by the image processing device, color of the initial fundus image on the basis of a difference between color values of a first reference region and a second reference region among the reference regions.

## Description

### [Technical Field]

The following description relates to a technique for standardizing color of a fundus image.

### [Background Art]

A fundus image is an image acquired by radiating illumination light into an eye and capturing light reflected from the fundus. The fundus image is used for diagnosing ocular lesions related to a macula, an optic disc, vascular conditions, and the like of the retina. For example, medical staff may determine whether there is a retina-related disease, such as diabetic retinopathy or the like, glaucoma, or an optic nerve disease on the basis of a fundus image. Further, an application recently emerged that allows artificial intelligence (Al) models to analyze fundus images to identify eye diseases.

### [Disclosure]

### [Technical Problem]

Since the amount of light entering a fundus varies depending on a patient's pupil size and media opacities, such as cataracts, and the light reflectance of the fundus (retina) varies from patient to patient, patients' fundus images may vary in brightness and color. Also, even the brightness or color of fundus images of the same patient may vary depending on the characteristics of examination equipment and environmental factors.

The technology described below is directed to providing a technique for standardizing fundus images with color deviations by uniformly correcting the fundus images.

### [Technical Solution]

In one general aspect, there is provided a method of standardizing a fundus image on the basis of an artery and a vein, the method including receiving, by an image processing device, an initial fundus image, determining, by the image processing device, reference regions in the initial fundus image, and correcting, by the image processing device, color of the initial fundus image on the basis of a difference between color values of a first reference region and a second reference region among the reference regions.

In another general aspect, there is provided a device for standardizing a fundus image on the basis of an artery and a vein, the device including an input device configured to receive an initial fundus image, a storage device configured to store a program for standardizing fundus image color, and a computational device configured to determine reference regions in the initial fundus image and correct color of the initial fundus image on the basis of a difference between color values of a first reference region and a second reference region among the reference regions.

### [Advantageous Effects]

According to the technology described below, a fundus image is standardized to aid medical staff in accurately diagnosing an eye disease. Further, the technology described below can be utilized as a technique for preprocessing input data in an application for analyzing fundus images.

### [Description of Drawings]

FIG. 1 is an example of a system for standardizing a fundus image.
FIG. 2 is an example of a process of standardizing a fundus image.
FIG. 3 is an example of a fundus image.
FIG. 4 is a set of examples of fundus images corrected on the basis of arterial regions.
FIG. 5 is an example of an application employing a standardized fundus image.
FIG. 6 is an example of an image processing device for standardizing a fundus image.

### [Modes of the Invention]

Although the technology described below can be diversely modified and can have several embodiments, specific embodiments will be illustrated in the accompanying drawings and described in detail. However, this is not intended to limit the technology described below to specific forms of implementation, and it should be understood that the technology described below includes all modifications, equivalents, or substitutions within the technical spirit and scope described below.

While terms such as "first," "second," "A," "B," and the like may be used in describing various components, the components are not limited by the terms, and the terms are used only for the purpose of distinguishing one component from another. For example, a first component may be named a second component without departing from the scope of the technology described below, and similarly, a second component may be named a first component. The term "and/or" includes a combination of a plurality of listed items or any of the plurality of listed items.

In terminology used in this specification, singular expressions are to be understood as including the plural unless the context clearly indicates otherwise, and terms such as "include" and the like refer to the described features, numbers, steps, operations, components, parts, or combinations thereof and do not preclude the possibility of the presence or addition of one or more other features, numbers, steps, operations, components, parts, or combinations thereof.

Before describing the drawings in detail, it should be noted that each element is distinguished by its main function. In other words, two or more elements to be described below may be joined as one element, or an element may be functionally subdivided into two or more elements. Also, each element to be described below may perform not only its main function but also some or all functions of other elements, and some main functions of elements may be fully performed by other elements.

In addition, in performing a method or an operating method, each process constituting the method may be performed in an order different from a specified order unless a specific order is explicitly described in the context. In other words, each process may be performed in the same order as a specified order, performed substantially simultaneously, or performed in reverse order.

The technology described below relates to a technique for standardizing a fundus image. An initial fundus image is captured using fundus examination equipment (a fundus camera). As described above, initial fundus images may vary in brightness and color depending on examination equipment, examination environments, and subject people. Fundus image standardization corresponds to a process of correcting color of a fundus image in accordance with a specific reference criterion.

It is assumed in the following description that an image processing device corrects a fundus image for standardization. The image processing device may be implemented as various devices for certain data processing. For example, the image processing device may be implemented as a personal computer (PC), a server on a network, a smart device, a chipset in which a dedicated program is embedded, or the like.

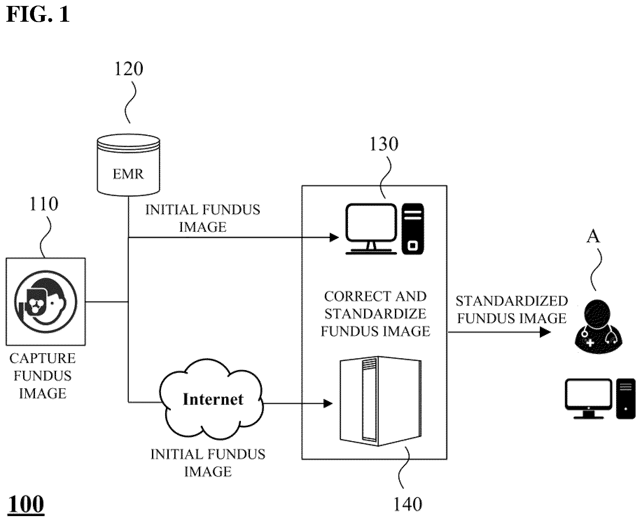

FIG. 1 is an example of a system 100 for standardizing a fundus image.

In the example of FIG. 1, an image processing device includes a computer terminal 130 and a server 140.

A fundus imaging device 110 generates a fundus image by photographing a patient's fundus. The fundus image generated by the fundus imaging device 110 may be stored in an electronic medical record (EMR) 120 or a separate database (DB).

In FIG. 1, a user A may uniformly standardize fundus images using the computer terminal 130. The computer terminal 130 may receive a fundus image from the fundus imaging device 110 or the EMR 120 via a wired or wireless network. In some cases, the computer terminal 130 may be a device physically connected to the fundus imaging device 110. The computer terminal 130 may correct color of the fundus image on the basis of arterial and/or venous regions in the fundus image. In this way, the computer terminal 130 may standardize the fundus image. A fundus image standardization process will be described below. The user A may check the standardized fundus image in the computer terminal 130.

The server 140 may receive a fundus image from the fundus imaging device 110 or the EMR 120. The server 140 may correct color of the fundus image on the basis of the arterial and/or venous region in the fundus image. In this way, the server 140 may standardize the fundus image. A fundus image standardization process will be described below. The user A may check the standardized fundus image.

The computer terminal 130 and/or the server 140 may store the standardized fundus image in the EMR 120 or the separate DB.

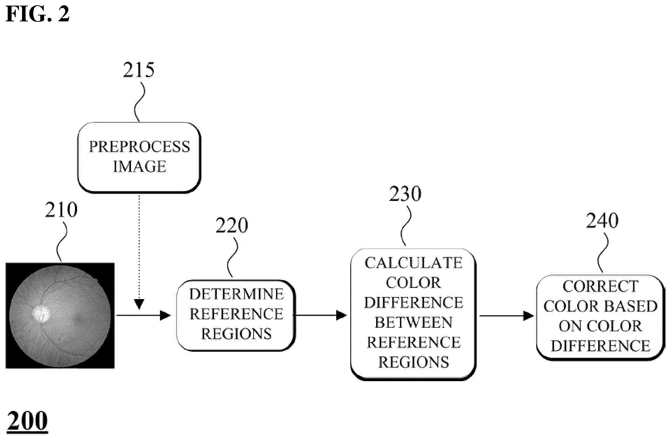

FIG. 2 is an example of a process 200 of standardizing a fundus image.

An image processing device receives an initial fundus image (210).

The image processing device may preprocess the initial fundus image uniformly (215). Preprocessing may include adjusting the resolution or size of the initial image, cropping an eye region from the initial fundus image, and the like.

The image processing device determines a reference region for standardizing color of the fundus image (220). A reference region is a region that will be used as a criterion for correcting color of the entire fundus image. As reference regions, an arterial region and a venous region checked in the fundus image may be used. Meanwhile, the image processing device may utilize an arterial region and a venous region in a partial region of the fundus image. Meanwhile, the arterial region may be referred to as a first reference region, and the venous region may be referred to as a second reference region.
(i) As reference regions, the image processing device may utilize an arterial region and a venous region set by a user through an interface device. (ii) The image processing device may determine a reference region through a general image processing process. The image processing device may determine a reference region using shapes and color of an arterial region and a venous region. Features of an arterial region and a venous region that will be reference regions will be described below. (iii) Otherwise, the image processing device may detect a reference region using a segmentation model. For example, the segmentation model may be a semantic segmentation model such as a U-shaped network (U-net), a fully connected network (FCN), or the like. In this case, the image processing device may determine reference regions (an arterial region and a venous region) for color comparison by inputting the initial fundus image to the trained segmentation model.

The segmentation model may distinguish between arterial regions and venous regions in the fundus image. Here, the segmentation model may crop a most effective region for standardization from among multiple arterial regions in the fundus image. Also, the segmentation model may crop a most effective region for standardization from among multiple venous regions in the fundus image.

The image processing device calculates a color difference between the arterial region and the venous region in the reference region (230). The image processing device calculates the color difference by comparing pixel values. The color difference may be calculated in various ways. The image processing device may determine the color difference on the basis of all red-green-blue (RGB) channels or a specific color channel. Alternatively, the image processing device may determine the color difference in a CbCr space by converting the RGB image into YCbCr.

The image processing device may correct color of the current initial fundus image on the basis of the color difference between the arterial region and the venous region (240).

The arterial region and the venous region have a red tone color. Therefore, the image processing device may correct the color of the entire fundus image such that the red color of the arterial region and/or the venous region has a certain color value.

Also, the colors of the arterial region and the venous region basically have a certain degree of difference. Therefore, the image processing device may correct the color of the entire image such that the color difference between the arterial region and the venous region in the current initial fundus image has a certain value (reference value). Here, the reference value may be a specific value or a specific range. In other words, the image processing device may correct the color of the initial fundus image such that the color difference between the arterial region and the venous region has a certain value or falls within a certain category. The image processing device may change a value of a specific color channel such that the color difference between the arterial region and the venous region is adjusted to the certain value. The image processing device may correct the color using a correction table that is provided in advance for each color channel, or a correction function.

Further, the image processing device may utilize an optic disc region as the reference region. The optic disc corresponds to the brightest region in the fundus image. Accordingly, the image processing device may adjust a brightness value of the entire fundus image on the basis of a brightness value of the optic disc. The image processing device may adjust the brightness of the entire fundus image such that the brightness value of the optic disc becomes a certain brightness value.

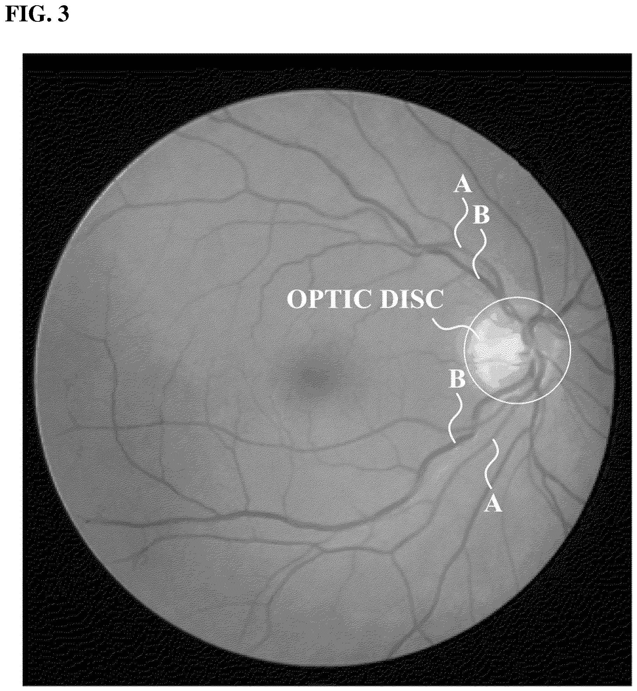

FIG. 3 is an example of a fundus image. In the image of FIG. 3, parts of arteries are indicated as A, and parts of veins are indicated as B. The arteries extend from the optic disc and branch off from the retina, and branched veins are gathered at the optic disc. The arteries and the veins are generally parallel to each other. The arteries and the veins have a difference which is observed in the image of FIG. 3. Arteries tend to be thinner and lighter in color than veins, and veins tend to be thicker and darker in color than arteries.

The color difference between arteries and veins results from a difference in the ratio of oxygenated hemoglobin (HbO₂) to deoxygenated hemoglobin (Hb). As is widely known, arteries have a higher ratio of oxygenated hemoglobin than veins. Further, arteries have thicker muscle within the vessel walls, and veins have thinner muscle, which is why veins appear thicker and have a darker red color than arteries.

In fundus images, arteries and veins have different degrees of oxygen saturation. With regard to this, there is a study result about the difference between arterial and venous oxygen saturation (Bahram Khoobehi et al, Retinal Arterial and Venous Oxygen Saturation Is Altered in Diabetic Patients, Invest Ophthalmol Vis Sci. 2013;54;7103-7106).

In the study, the degrees of oxygen saturation of arteries and veins were measured from the retinas of normal people, diabetics without diabetic retinopathy, diabetics with mild-to-moderate diabetic retinopathy, and diabetics with severe diabetic retinopathy. In brief, (i) in normal people, arterial blood oxygen saturation was 92.3±4.2%, and venous blood oxygen saturation was 57.2±6.0%, resulting in a difference of 35.2%. (ii) In diabetics without diabetic retinopathy, arterial blood oxygen saturation was 96.3±8.6%, and venous blood oxygen saturation was 58.7±7.5%, resulting in a difference of 37.6%. (iii) In diabetics with mild-to-moderate diabetic retinopathy, arterial blood oxygen saturation was 97.7±5.8%, and venous blood oxygen saturation was 61.1±7.6%, resulting in a difference of 36.6%. (iv) In diabetics with sever diabetic retinopathy, arterial blood oxygen saturation was 102±10.2%, and venous blood oxygen saturation was 66.8±8.4%, resulting in a difference of 35.2%. In other words, differences in oxygen saturation between arterial and venous blood in retinas are relatively constant irrespective of eye disease. Also, arterial blood oxygen saturation ranges from 92% to 103.6%, and venous blood oxygen saturation ranges from 57.2% to 66.8%.

The existing study results that arteries and veins of a retina separately have certain oxygen saturation distributions and that the difference in oxygen saturation between arteries and veins is constant irrespective of subject people lead to a conclusion that the difference between the color of the arteries and veins in fundus images is also constant. Therefore, as described in FIG. 2, the image processing device can correct a fundus image on the basis of colors of arterial regions and/or venous regions in the fundus image or a color difference between the arterial regions and the venous regions. The image processing device normalizes or standardizes an input fundus image(s) uniformly.

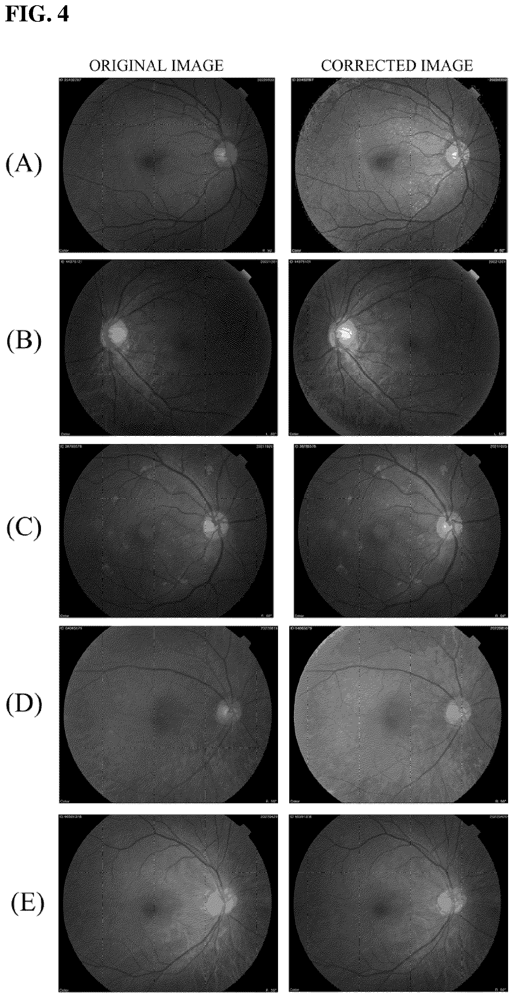

The researcher corrected color of fundus images collected at his or her affiliated institution on the basis of arterial regions. FIG. 4 is a set of examples of fundus images corrected on the basis of arterial regions. Assuming that patients' arterial blood has oxygen saturation of 95% to 100%, the researcher corrected the fundus images on the basis of color information of four spots in retinal veins. FIG. 4 shows correction results of five images A, B, C, D, and E. Color of the corrected fundus images shows uniformly standardized results.

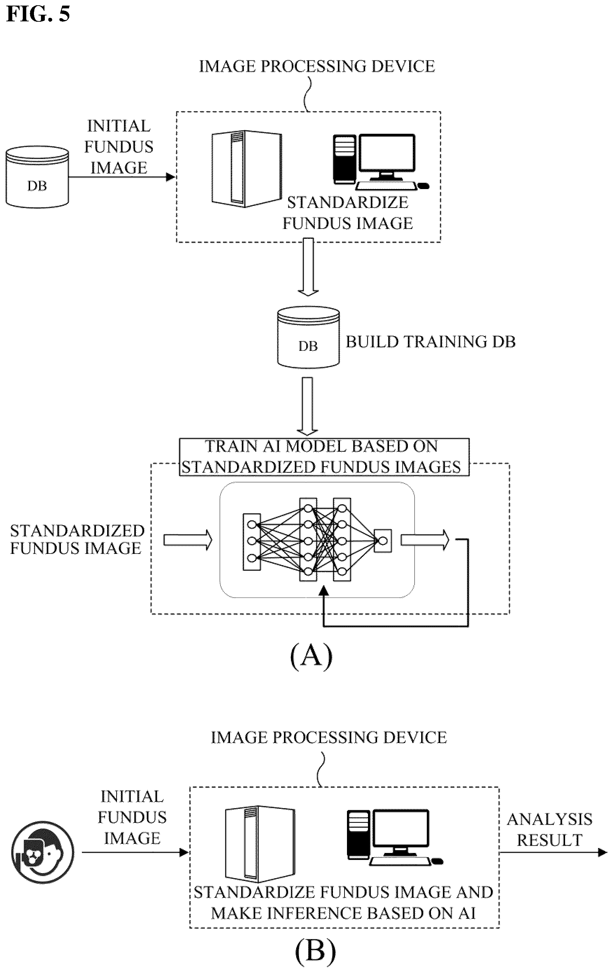

FIG. 5 is an example of an application employing a standardized fundus image. FIG. 5 is an example of building a learning model or an artificial intelligence (AI) model that analyzes a standardized fundus image. The AI model may be a model that analyzes a patient's fundus image to predict or diagnose an eye disease.

FIG. 5A is an example of a process of training an AI model. An image processing device receives initial fundus images generated by a fundus examination apparatus. Here, the image processing device may be a separate learning device. The image processing device may receive initial fundus images from a separate DB. The image processing device standardizes the initial fundus images through the above-described process. The image processing device may store the standardized fundus images in a training DB. The image processing device trains the AI model using the standardized fundus images. The image processing device repeats a process of inputting the standardized fundus images to the AI model and comparing values output by the AI model with previously known label values to update parameters of the AI model. In this way, the image processing device prepares the AI model for predicting eye diseases.

FIG. 5B is an example of a process of predicting an eye disease using the trained AI model. The image processing device receives an initial fundus image captured by a fundus imaging device. The image processing device standardizes the initial fundus image. The image processing device inputs the standardized fundus image to the AI model trained through FIG. 4A. The image processing device makes an inference about an ocular lesion using the AI model. The image processing device may derive an analysis result about whether there is an eye disease in accordance with a value (possibility value) output by the AI model.

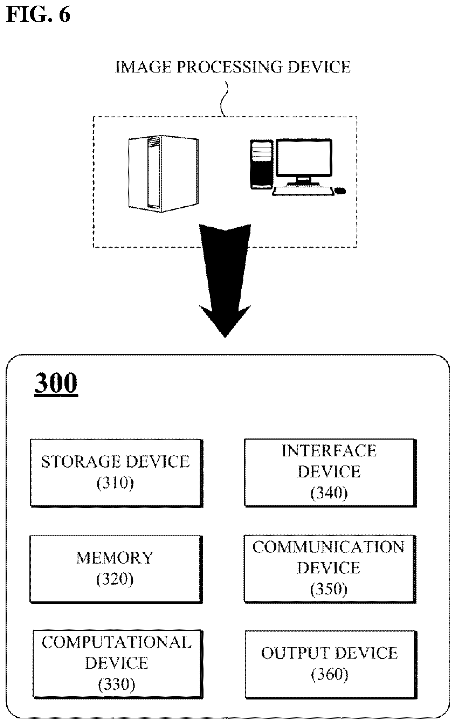

FIG. 6 is an example of an image processing device 300 for standardizing a fundus image. The image processing device 300 corresponds to the above-described image processing devices 130 and 140 (see FIG. 1). The image processing device 300 may be physically implemented in various forms. For example, the image processing device 300 may have the form of a computer device, such as a PC, a server on a network, a chipset dedicated to data processing, or the like.

The image processing device 300 may include a storage device 310, a memory 320, a computational device 330, an interface device 340, a communication device 350, and an output device 360.

The storage device 310 may store an initial fundus image generated by fundus examination equipment.

The storage device 310 may store code or a program for standardizing initial fundus images.

The storage device 310 may store reference information (a criterion value for color difference between an artery and a vein, a color criterion value (reference value) for arterial regions, a color criterion value for venous regions, a brightness criterion value for optic disc regions, and the like) for color correction.

The storage device 310 may store a program or a learning model (segmentation model) that identifies a reference region for fundus image standardization.

The storage device 310 may store standardized fundus images.

The memory 320 may store data, information, and the like generated in a process in which the image processing device 300 standardizes (corrects) a fundus image.

The interface device 340 is a device for receiving a certain external command and data. The interface device 340 may receive an initial fundus image from an input device or an external storage device physically connected thereto. The interface device 340 may receive setting information for a reference region in the initial fundus image. The interface device 340 may transfer a standardized fundus image to an external object.

The communication device 350 is an element that receives and transmits certain information via a wired or wireless network. The communication device 350 may receive an initial fundus image from an external object. The communication device 350 may receive setting information for a reference region in the initial fundus image. The communication device 350 may transmit a standardized fundus image to an external object such as a user terminal.

The interface device 340 and the communication device 350 are elements that exchange certain data with a user or another physical object, and thus may be collectively named an input/output device. In terms of an information or data input function, the interface device 340 and the communication device 350 may be referred to as input devices.

The output device 360 is a device that outputs certain information. The output device 360 may output an interface, an initial fundus image, a standardized fundus image, and the like required for a data processing process.

The computational device 330 may use the code, the program, or the learning model to perform the following operation.

The computational device 330 may preprocess the initial fundus image uniformly. The preprocessing may be any one operation among changing the resolution or size, cropping a certain region, removing noise, and the like.

The computational device 330 may determine a reference region on which to base color correction in the initial fundus image. The computational device 330 may determine a reference region on the basis of information input by a user. Also, the computational device 330 may automatically determine a reference region on the basis of an image processing program. Further, the computational device 330 may identify a reference region using a trained segmentation model. As described above, reference regions include an arterial region (first reference region) and a venous region (second reference region). Reference regions may be a partial region of arteries and a partial region of veins in the fundus image. In addition, reference regions may further include an optic disc region (third reference region).

The computational device 330 may compare a brightness value of the optic disc region with a previously known brightness reference value and adjust a brightness value of the entire fundus image such that the current brightness value of the optic disc region is identical or close to the brightness reference value.

The computational device 330 may correct color of the entire fundus image on the basis of color of an arterial region and/or a venous region among reference regions. In other words, the computational device 330 may correct the color of the entire fundus image by correcting color such that a color criterion value (color reference value) generally expected in arterial regions is adjusted to be identical or similar to a color value of the current arterial region.

The computational device 330 may correct the color of the fundus image on the basis of an arterial region and a venous region in the optic disc region. In other words, the computational device 330 may correct the color of the entire fundus image on the basis of color of the arterial region and color of the venous region. The image processing device may correct the color of the fundus image on the basis of various criteria. (i) The computational device 330 may correct the color of the fundus image such that a difference between the color of the arterial region and the color of the venous region becomes a certain reference value. (ii) The computational device 330 may correct the color of the fundus image such that the difference between the color of the arterial region and the color of the venous region falls within a certain reference range. (iii) The computational device 330 may determine a color correction value of the fundus image by referring to a table provided in advance on the basis of the current color of the arterial region and the current color of the venous region. Here, the color correction value may include values of color channels for correction.

The computational device 330 may determine a color difference by comparing a specific arterial region with a specific venous region in the reference region. The computational device 330 may correct all colors such that the color difference between the arterial region and the venous region becomes a previously known reference value. The computational device 330 may adjust a color value of a specific color channel or a plurality of color channels such that the difference between the arterial region and the venous region becomes identical or close to the known reference value. In this way, the computational device 330 may normalize or standardize color of the input fundus image uniformly.

Further, the computational device 330 may input the standardized fundus image to a pretrained AI model to make an inference about an eye disease.

The computational device 330 may be a device that processes data and certain computation, such as a processor, an application processor (AP), or a chip into which a program is embedded.

The above-described method of processing a medical image or standardizing a fundus image may be implemented as a program (or application) including an algorithm that is executable in a computer. The program may be stored and provided in a transitory or non-transitory computer readable medium.

The non-transitory computer readable medium is a medium that stores data semi-permanently and is readable by a device, rather than a medium that stores data for a short time period, such as a register, a cache, a memory, or the like. Specifically, the foregoing various applications or programs may be stored and provided in a non-transitory computer readable medium such as a compact disc (CD), a digital video disc (DVD), a hard disk, a Blu-ray disc, a Universal Serial Bus (USB) device, a memory card, a read-only memory (ROM), a programmable read only memory (PROM), an erasable PROM (EPROM), an electrically EPROM (EEPROM), a flash memory or the like.

The transitory computer readable medium is any of various random access memories (RAMs) such as a static RAM (SRAM), a dynamic RAM (DRAM), a synchronous DRAM (SDRAM), a double data rate (DDR) SDRAM, an enhanced SDRAM (ESDRAM), a SyncLink DRAM, and a direct Rambus RAM (DRRAM).

The present embodiments and the accompanying drawings clearly illustrate only a part of the technical spirit included in the above-described technology, and it will be apparent that all modified examples and specific embodiments that can be readily inferred by those of ordinary skill in the art within the scope of the technical spirit included in the description and drawings of the above-described technology fall within the scope of the above-described technology.

## Claims

1. A method of standardizing a fundus image on the basis of an artery and a vein, the method comprising:
receiving, by an image processing device, an initial fundus image;
determining, by the image processing device, reference regions in the initial fundus image; and
correcting, by the image processing device, color of the initial fundus image on the basis of a difference between color values of a first reference region and a second reference region among the reference regions.

2. The method of claim 1, wherein the first reference region is an arterial region, and
the second reference region is a venous region.

3. The method of claim 1, wherein the image processing device corrects the color of the initial fundus image such that the difference between the color values becomes a previously known reference value or falls within a previously known reference range.

4. The method of claim 1, further comprising correcting, by the image processing device, the color of the initial fundus image to have a previously known color reference value on the basis of any one of the first reference region and the second reference region.

5. The method of claim 1, wherein the reference regions are set using information input by a user, or extracted from the initial fundus image using a separate learning model.

6. An image processing device for standardizing a fundus image on the basis of an artery and a vein, the image processing device comprising:
an input device configured to receive an initial fundus image;
a storage device configured to store a program for standardizing fundus image color; and
a computational device configured to determine reference regions in the initial fundus image and correct color of the initial fundus image on the basis of a difference between color values of a first reference region and a second reference region among the reference regions.

7. The image processing device of claim 6, wherein the first reference region is an arterial region, and
the second reference region is a venous region.

8. The image processing device of claim 6, wherein the computational device corrects the color of the initial fundus image to have a previously known color reference value on the basis of any one of the first reference region and the second reference region.

9. The image processing device of claim 6, wherein the computational device corrects the color of the initial fundus image such that the difference between the color values becomes a previously known reference value or falls within a previously known reference range.
